# FASCICULE DE BREVET EUROPEEN

(11) **EP 0 229 046 B1**
(45) Date de publication et mention de la délivrance du brevet: **04.05.1994**
(21) Numéro de dépôt: 85902946.4
(22) Date de dépôt: 17.06.1985
(51) Int. Cl.: C12N 15/11, C12P 21/00, C07K 15/00, G01N 33/48, A61K 37/02, A61K 39/00, A61K 39/29

(54) **PROCEDE D'OBTENTION D'ADN, ARN, PEPTIDES, POLYPEPTIDES OU PROTEINES, PAR UNE TECHNIQUE DE RECOMBINAISON D'ADN**
VERFAHREN ZUM ERHALTEN VON DNS, RNS, PEPTIDEN, POLYPEPTIDEN ODER PROTEINEN DURCH DNS-REKOMBINANT-VERFAHREN
METHOD FOR OBTAINING DNA, RNA, PEPTIDES, POLYPEPTIDES OR PROTEINS BY MEANS OF A DNA RECOMBINANT TECHNIQUE

(30) Priorité: 30.03.1985 CH 1379/85
(43) Date de publication de la demande: 22.07.1987
(60) Demande divisionnaire: 93116225.9
(73) Titulaire: BALLIVET, Marc, 1208 Genève (CH); KAUFFMAN, Stuart Alan, Bryn Mawr, PA 12010 (US)
(72) Inventeur: BALLIVET, Marc, 1208 Genève (CH); KAUFFMAN, Stuart Alan, Bryn Mawr, PA 12010 (US)
(74) Mandataire: Kovacs, Paul
(86) Numéro de dépôt international: CH8500099
(87) Numéro de publication internationale: WO8605803

(56) Documents cités:
- DE-A- 3 246 071
- DE-A- 3 300 632
- DE-A- 3 303 173
- T212/88, T60/89, T580/88, T226/85; Science, 1990, vol. 249, 404; Science, 1990, vol. 249, 386; Science, 1990, vol. 249, 505; Nature, 1990, vol. 346, 818; Genome, 1989, vol. 31, 112.
- Chemical Abstracts, volume 84, no. 5, 2 février 1976, Columbus, Ohio (US) G.W. Hoffmann: "Stochastic theory of the origin of the genetic code", voir page 136, abrégé 26960x.

## Description

La présente invention a pour objet un procédé d'obtention d'ADN, ARN, peptides, polypeptides ou protéines, au moyen de cellules-hôtes modifiées contenant des gènes capables d'exprimer ces ARN, peptides, polypeptides ou protéines, c'est-à-dire en utilisant une technique de recombinaison d'ADN.

L'invention vise notamment à produire des gènes ou fragments de gènes stochastiques, de façon à permettre l'obtention simultanée, après transcription et traduction de ces gènes, d'un très grand nombre (de l'ordre de dix mille au moins) de protéines complètement nouvelles ou hybrides de protéines connues, en présence de cellules-hôtes (souches bactériennes ou eucaryotes) contenant les gènes respectivement capables d'exprimer ces protéines et d'effectuer ensuite une sélection ou criblage parmi lesdites souches, en vue de déterminer celles qui produisent des protéines présentant des propriétés désirées, par exemple des propriétés structurelles enzymatiques, catalytiques, antigéniques, pharmacologiques, ou des propriétés de ligand, et plus généralement, des propriétés chimiques, biochimiques, biologiques, etc.

L'invention a également pour but de permettre l'obtention de séquences d'ADN ou d'ARN présentant des propriétés utilisables, notamment des propriétés chimiques, biochimiques ou biologiques.

On comprend donc que l'invention est susceptible de recevoir de très nombreuses applications, dans des domaines très variés de la science, de l'industrie et de la médecine.

Le procédé de production de peptides ou polypeptides selon l'invention est caractérisé en ce que l'on produit simultanément, au sein d'un même milieu, des gènes au moins partiellement composés de polynucléotides synthétiques stochastiques, que l'on introduit les gènes ainsi obtenus dans des cellules-hôtes, que l'on cultive simultanément les souches indépendantes de cellules-hôtes modifiées renfermant ces gènes, de manière à cloner les gènes stochastiques et à provoquer la production des protéines exprimées par chacun de ces gènes stochastiques, que l'on effectue le criblage et/ou la sélection des souches de cellules-hôtes modifiées de façon à identifier les souches produisant des peptides ou polypeptides ayant au moins une propriété donnée, que l'on isole les souches ainsi identifiées et qu'on les cultive de façon à produire au moins un peptide ou polypeptide ayant ladite propriété.

Conformément à un premier mode de mise en oeuvre de ce procédé, on produit les gènes par copolymérisation stochastique des quatre sortes de deoxyphosphonucléotides A, C, G et T à partir des deux extrémités d'un vecteur d'expression préalablement linéarisé, puis formation d'extrémités cohésives de façon à former un premier brin d'ADN stochastique, constitué d'une molécule du vecteur d'expression possédant deux séquences stochastiques dont les extrémités 3' sont complémentaires, suivie de la synthèse du second brin de cet ADN stochastique.

Selon un deuxième mode de mise en oeuvre, on produit les gènes par copolymérisation stochastique d'oligonucléotides sans extrémités cohésives, de façon à former des fragments d'ADN stochastiques, suivie de la ligation de ces fragments à un vecteur d'expression préalablement linéarisé.

Le vecteur d'expression peut être un plasmide, notamment un plasmide bactérien. D'excellents résultats ont été obtenus en utilisant, comme vecteur d'expression, le plasmide pUC8.

Le vecteur d'expression peut également être un ADN viral ou un hybride de plasmide et d'ADN viral.

Les cellules-hôtes peuvent être des cellules procaryotes telles que les cellules HB 101 et C 600, ou des cellules eucaryotes.

Lorsque l'on procède conformément au deuxième mode de mise en oeuvre susmentionné, on peut utiliser des oligonucléotides formant un groupe d'octamères palindromiques.

Des résultats particulièrement bons sont obtenus en utilisant le groupe d'octamères palindromiques suivant :
5' GGAATTCC 3'
5' GGTCGACC 3'
5' CAAGCTTG 3'
5' CCATATGG 3'
5' CATCGATG 3'
On peut également utiliser des oligonucléotides formant un groupe d'heptamères palindromiques.

De très bons résultats sont obtenus en utilisant le groupe d'heptamères palindromiques suivant :
5' XTCGCGA 3'
5' XCTGCAG 3'
5' RGGTACC 3'
où X = A, G, C ou T et R = A ou T.

Conformément à un mode de mise en oeuvre particulièrement avantageux, l'on isole et purifie l'ADN transformant des plasmides provenant d'une culture de souches indépendantes de cellules-hôtes modifiées obtenues en procédant de la manière spécifiée ci-dessus, puis l'on provoque la coupure de l'ADN purifié au moyen d'au moins un enzyme de restriction correspondant à un site spécifique de coupure enzymatique présent dans ces octamères ou heptamères palindromiques mais absent du vecteur d'expression utilisé, cette coupure étant suivie de l'inactivation de l'enzyme de restriction, et l'on traite ensuite simultanément l'ensemble des fragments d'ADN stochastiques linéarisés, ainsi obtenus, par la T4 DNA ligase, de façon à créer un nouvel ensemble d'ADN contenant des séquences stochastiques nouvelles, ce nouvel ensemble pouvant donc contenir un nombre de gènes stochastiques supérieur au nombre de gènes de l'ensemble initial, et on utilise ce nouvel ensemble d'ADN transformant pour modifier des cellules-hôtes et cloner les gènes, et, finalement, on crible et/ou on sélectionne et on isole les nouvelles souches de cellules-hôtes transformées et finalement, on les cultive de façon à produire au moins un peptide ou polypeptide, par exemple une protéine nouvelle.

La propriété servant de critère de sélection des souches de cellules-hôtes peut être l'aptitude des peptides ou polypeptides, produits par cette souche, à catalyser une réaction chimique donnée, notamment l'aptitude à modifier sélectivement l'activité catalytique d'un polypeptide.

Ladite propriété peut être, également, l'aptitude du peptide ou polypeptide à se lier à un ligand donné.

Selon une variante du procédé, l'on identifie et isole les souches de cellules-hôtes modifiées produisant les peptides ou polypeptides ayant la propriété désirée par chromatographie d'affinité sur anticorps correspondant à une protéine exprimée par la partie naturelle de l'ADN hybride.

Par exemple, dans le cas où la partie naturelle de l'ADN hybride contient un gène exprimant la β-galactosidase, l'on peut avantageusement identifier et isoler lesdites souches de cellules-hôtes modifiées par chromatographie d'affinité sur anticorps anti-β-galactosidase.

Après expression et purification des peptides ou polypeptides hybrides, on peut séparer et isoler leurs parties nouvelles.

Avantageusement, conformément à un mode de mise en oeuvre du procédé selon l'invention, les cellules-hôtes consistent en bactéries du genre Escherichia coli dont le génome ne contient ni le gène naturel exprimant la β-galactosidase, ni le gène EBG, c'est-à-dire des bactéries E. coli (Z⁻, EBG⁻), on cultive les cellules-hôtes modifiées en présence du milieu X-gal et de l'inducteur IPTG, que l'on détecte, dans le milieu de culture, les clones positifs pour la fonction β-galactosidase, et, finalement, on transplante ensuite cet ADN dans une souche de cellules-hôtes appropriée à la culture en grande quantité en vue de la production industrielle d'au moins un peptide ou polypeptide.

La propriété servant de critère de sélection des souches de cellules-hôtes transformées peut également être l'aptitude, des polypeptides ou protéines, produites par culture de ces souches, à se lier à un composé donné.

Ce composé peut être, notamment, avantageusement choisi parmi les peptides, les polypeptides et les protéines.

D'autre part, ledit composé peut également être choisi parmi les séquences d'ADN et d'ARN.

L'invention a également pour objet un procédé de production d'ADN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on effectue un criblage et/ou une sélection de cet ensemble, de façon à identifier les cellules hôtes qui renferment dans leur génome des séquences stochastiques d'ADN présentant au moins une propriété désirée, et l'on isole finalement l'ADN à partir de cultures des cellules-hôtes ainsi identifiées.

L'invention a, en outre, pour objet un procédé de production d'ARN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on cultive simultanément les souches indépendantes de cellules-hôtes modifiées ainsi produites, que l'on effectue un criblage et/ou une sélection de cet ensemble, de façon à identifier les cellules-hôtes qui renferment des séquences stochastiques d'ARN présentant au moins une propriété désirée, et que l'on isole l'ARN à partir de cultures de cellules-hôtes ainsi identifiées.

Ladite propriété peut être, avantageusement, l'aptitude à se lier à un composé donné, qui peut être, par exemple, un peptide, un polypeptide ou une protéine, ou bien l'aptitude à catalyser une réaction chimique donnée, ou encore la propriété d'avoir la fonction d'un ARN de transfert.

On va maintenant décrire, plus en détails, le procédé selon l'invention, ainsi que certaines de ses applications, en se référant à des exemples, non limitatifs, de mise en oeuvre.

Tout d'abord, on va décrire des modes opératoires particulièrement avantageux pour effectuer la synthèse de gènes stochastiques et l'introduction de ces gènes dans des bactéries, de manière à produire des souches de bactéries transformées.

### I) Synthèse directe sur un vecteur d'expression

### a) Linéarisation du vecteur

30 µg (c'est-à-dire approximativement 10¹³ molécules) du vecteur d'expression pUC8 sont linéarisés par incubation pendant 2 heures à 37°(avec 100 unités de l'enzyme de restriction PstI dans une volume de 300 µl du tampon standard approprié. Le vecteur linéarisé est traité au phénol-chloroforme puis précipité à l'éthanol, repris dans un volume de 30 µl et chargé sur un gel d'agarose à 0,8 %, en milieu standard TEB. Après migration dans un champ de 3V/cm pendant trois heures, le vecteur linéaire est électro-élué, précipité à l'éthanol et repris dans 30 µl d'eau.

### b) Synthèse stochastique par l'enzyme

### Terminal-Transférase (TdT)

On fait réagir 30 µg du vecteur linéarisé avec 30 unités de TdT dans 300 µl du tampon approprié, pendant deux heures, à 37°C, en présence de 1mM de dGTP, 1mM de dCTP, 0,3mM de dTTP et 1mM de dATP: On choisit une concentration plus basse de dTTP, dans le but de diminuer la fréquence des codons "stop" dans l'ARN messager correspondant. Un résultat semblable, quoique moins favorable, peut être obtenu en utilisant une concentration plus basse pour le d'ATP que pour les autres deoxynucléotides triphosphates.Le progrès de la réaction de polymérisation sur l'extrémité 3' des sites Pst I est suivi par l'analyse sur gel de parties aliquotes prélevées en cours de réaction.

Lorsque la réaction atteint ou dépasse une valeur moyenne de 300 nucléotides par extrémité 3', elle est interrompue et les nucléotides libres sont séparés du polymère par précipitation différentielle ou par passage sur tamis moléculaire du type Biogel P60. Après concentration par précipitation à l'éthanol, le polymère est soumis séquentiellement à une polymérisation supplémentaire par TdT en présence de dATP d'abord, puis de dTTP. Ces deux dernières réactions sont séparées par une filtration sur gel et conduites pendant des temps courts (30 secondes à 3 minutes), de manière à aboutir à l'addition séquentielle de 10-30A, suivis de 10-30 T à l'extrémité 3' des polymères.

### c) Synthèse du second brin de l'ADN stochastique

Chaque molécule de vecteur possède, à l'issue des opérations précédentes, deux séquences stochastiques dont les extrémités 3' sont complémentaires. Le mélange de polymères est alors incubé dans des conditions favorisant l'hybridation de ces extrémités complémentaires (150 mM Nacl, 10 mM Tris-HCl, pH 7,6, 1 mM EDTA, à 65°C, pendant 10 minutes, puis abaissement de la température jusqu'à 22°C, à raison de 3 à 4°C/heure). Les polymères hybridés sont alors soumis à l'action de 60u du grand fragment de la polymérase I (Klenow) en présence des quatre nucléotides triphosphates (200mM) à 4°C, pendant deux heures. Cette étape réalise la synthèse du second brin à partir de l'extrémité 3' des polymères hybridés. Les molécules résultant de cette synthèse directe à partir d'un vecteur linéarisé sont alors utilisées pour transformer des cellules compétentes.

### d) Transformation des souches compétentes

100 à 200 ml de cellules compétentes HB101 ou C600, à la concentration de 10¹⁰ cellules/ml, sont incubés avec la préparation d'ADN stochastique, en présence de 6mM CaCl₂, 6 mM Tris-Hcl, pH8, 6 mM MgCl₂, pendant 30 minutes, à 0°C. Un choc thermique de 3 minutes à 37°C est imposé au mélange, suivi par l'addition de 400-800 ml de milieu de culture NZY, sans antibiotique. La culture transformée est incubée à 37°C pendant 60 minutes, puis diluée à 10 litres par addition de milieu NZY contenant 40 µg/ml d'ampicilline. Après 3 à 5 heures d'incubation à 37°C, la culture amplifiée est soumise à une centrifugation, et le culot des cellules transformées est lyophilysé et conservé à -70°C. Une telle culture comprend de 3 x 10⁷ à 10⁸ transformants indépendants, contenant chacun un gène stochastique unique inséré dans un vecteur d'expression.

### II) Synthèse de gènes stochastiques à partir d'oligonucléotides sans extrémités cohésives

Ce mode opératoire est fondé sur le fait que la polymérisation d'oligonucléotides palindromiques judicieusement choisis permet d'assembler des gènes stochastiques ne comportant aucun codon "stop" dans chacun des six cadres de lecture possibles, tout en assurant une représentation équilibrée de triplets spécifiant tous les acides aminés. Par ailleurs, et pour éviter la répétition de motifs de séquences dans la protéine résultante, les oligonucléotides penvent comporter un nombre de bases qui n'est pas un multiple de trois. L'exemple suivant décrit l'utilisation d'une combinaison possible d'oligonucléotides qui remplit ces critères :

### a) Choix d'un groupe d'octamères

Le groupe d'oligonucléotides suivant :
5' GGAATTCC 3'
5' GGTCGACC 3'
5' CAAGCTTG 3'
5' CCATATGG 3'
5' CATCGATG 3'
est composé de 5 palindromes (donc autocomplémentaires) dont il est facile de vérifier que leur polymérisation stochastique n'engendre pas de codon "stop" et spécifie tous les acides aminés.

Bien entendu, on pourrait également utiliser d'autres groupes d'octamères palindromiques n'engendrant pas de codons "stop" et spécifiant tous les acides aminés des polypeptides. On comprendra également que l'on pourrait utiliser des groupes d'octamères non palindromiques à condition d'employer aussi leurs compléments par la formation de segments bicaténaires.

### b) Assemblage d'un gène stochastique à partir d'un groupe d'octamères

On fait réagir un mélange comportant 5 µg de chacun des oligonucléotides indiqués ci-dessus (préalablement phosphorylés en 5' par un procédé connu en soi) dans un volume de 100 µl contenant 1 mM ATP, 10 % de polyéthylèneglycol et 100 u de T₄ DNA ligase, dans le tampon approprié, à 13°C, pendant six heures. Cette étape effectue la polymérisation stochastique des oligomères sous forme bi-caténaire, sans extrémités cohésives. On isole alors par passe sur tamis moléculaire (Biogel P60) les polymères résultant de l'assemblage de 20 à 100 oligomères. Après concentration, cette fraction est à nouveau soumise à la polymérisation catalysée par la T4 DNA ligase dans les conditions décrites ci-dessus. On isole alors, comme décrit plus haut, les polymères résultant de l'assemblage d'au moins 100 oligomères.

### c) Préparation du plasmide-hôte

Le vecteur d'expression pUC8 est linéarisé par l'enzyme Sma I dans le tampon approprié, comme décrit ci-dessus. Le vecteur linéarisé par l'enzyme Sma I ne comporte pas d'extrémités cohésives. Le vecteur linéarisé est alors traité par la phosphatase alcaline d'intestin de veau (CIP) à raison d'une unité par µg de vecteur dans le tampon approprié, à 37°C, pendant 30 minutes. L'enzyme CIP est alors inactivée par deux extractions successives au phénol-chloroforme. Le vecteur linéarisé et déphosphorylé est précipité à l'éthanol puis repris dans l'eau à 1 mg/ml.

### d) Ligation des gènes stochastiques au vecteur

Des quantités équimolaires de vecteur et de polymère sont mélangées et incubées en présence de 1000 u de T4 DNA ligase, 1 mM ATP, 10 % de polyéthylène glycol dans le tampon approprié, pendant 12 heures, à 13°C. Cette étape réalise la ligation des polymères dans le vecteur d'expression et engendre des molécules bicaténaires, circulaires et, par conséquent, transformantes.

### Transformation des souches compétentes

On procède à la transformation des souches compétentes de la manière précédemment décrite.

### III) Assemblage d'un gène stochastique à partir d'un groupe d'heptamères

Ce mode opératoire se distingue de celui qui vient d'être décrit par le fait qu'il utilise des heptamères palindromiques comportant une extrémité cohésive variable, au lieu d'octamères. Il présente l'avantage de permettre l'assemblage de séquences stochastiques comportant une plus faible proportion de motifs identiques.

### a) Choix d'un groupe d'heptamères

On peut utiliser, par exemple, les 3 heptamères palindromiques suivants :
5' XTCGCGA 3'
5' XCTGCAG 3'
5' RGGTACC 3'
pour lesquels X = A, G, C ou T et R = A ou T et dont la polymérisation ne peut engendrer de codons "stop" et comporte des triplets spécifiant tous les acides aminés.

Bien entendu, on pourrait également utiliser tout autre groupe d'heptamères remplissant ces mêmes conditions.

### b) Polymérisation d'un groupe d'heptamères

Cette polymérisation s'effectue exactement de la manière précédemment décrite dans le cas des octamères.

### c) Elimination des extrémités cohésives

Les polymères ainsi obtenus comportent une base non appariée à leur deux extrémités 5'. Il est donc nécessaire d'additionner la base complémentaire aux extrémités 3' correspondantes. Ceci s'effectue de la manière suivante : on fait réagir 10 µg de polymères double brin avec 10 u d'enzymes de Klenow, en présence des quatre déoxynucléotidephosphates (200 mM) dans un volume de 100 µl, à 4°C, pendant 60 minutes. L'enzyme est inactivée par extraction au phénol-chloroforme et les polymères sont débarrassés des nucléotides libres résiduels par précipitation différentielle. Les polymères sont alors ligasés au plasmide-hôte (préalablement linéarisé et déphosphorylé) en procédant de la manière décrite ci-dessus.

Il est à remarquer que les deux derniers modes opératoires qui viennent d'être décrits utilisent des octamères ou heptamères palindromiques qui constituent des sites spécifiques d'enzymes de restriction. Ces sites sont absents pour la plupart du vecteur d'expression pUC8. Il est donc possible d'augmenter considérablement la complexité d'une préparation initiale de gènes stochastiques en procédant de la manière suivante : on prépare l'ADN des plasmides provenant d'une culture de 10⁷ transformants indépendants obtenus par l'un des deux derniers modes opératoires décrits ci-dessus. A cet ADN purifié, on fait alors subir une digestion partielle par l'enzyme de restriction Cla I (mode opératoire II) ou par l'enzyme de restriction Pst I (mode opératoire III). Après inactivation de l'enzyme, l'ADN partiellement digéré est traité à la T4 DNA ligase, ce qui a pour effet de créer un nombre extrêmement grand de séquences nouvelles qui conservent les propriétés fondamentales des séquences initiales. Ce nouvel ensemble de séquences stochastiques est alors utilisé pour transformer des cellules compétentes.

Par ailleurs, les gènes stochastiques clonés en procédant conformément aux modes opératoires II et III peuvent être excisés intacts du vecteur d'expression pUC8 en utilisant les sites de restriction propres au vecteur de clonage et non représenté dans l'ADN stochastique.

La recombinaison au sein des gènes stochastiques engendrés par les deux derniers modes opératoires qui viennent d'être décrits, recombinaison qui résulte de l'homologie interne et des motifs de récurrence, fournit une importante méthode additionnelle de mutagenèse in vivo des séquences codantes. Il en résulte une augmentation du nombre des gènes nouveaux qui peuvent être examinés.

Finalement, pour tout processus de génération de gènes synthétiques nouveaux, on peut utiliser de nombreuses techniques usuelles de modification des gènes in vivo ou in vitro, telles que changement du cadre de lecture, inversion des séquences par rapport à leur promoteur, ou utilisation de souches-hôtes exprimant un ou plusieurs tRNA suppresseurs.

En se référant à la partie de la description qui précède, on comprendra qu'il est possible de construire in vitro un nombre extrêmement grand (par exemple, supérieur à un milliard) de gènes différents, par polymérisation enzymatique de nucléotides et d'oligonucléotides. Cette polymérisation s'effectue selon un processus stochastique déterminé par les concentrations respectives des nucléotides ou oligonucléotides présents dans le milieu de réaction.

Comme indiqué ci-dessus, deux méthodes peuvent être utilisées pour cloner de tels gènes (ou séquences codantes) : la polymérisation peut s'effectuer directement sur un vecteur de clonage d'expression, préalablement linéarisé, ou bien on peut choisir de procéder séquentiellement à la polymérisation puis à la ligation des polymères au vecteur de clonage et d'expression.

Dans les deux cas, on procède ensuite à la transformation ou à la transfection de cellules bactériennes compétentes (ou de cellules en culture). Cette étape réalise le clonage des gènes stochastiques dans des cellules vivantes où ils sont indéfiniment propagés et exprimés.

Bien entendu, outre les modes opératoires qui viennent d'être décrits, on pourrait également employer toute autre méthode appropriée pour la synthèse de séquences stochastiques. En particulier, on pourrait procéder à la polymérisation, par voie biochimique, d'oligomères monocaténaires d'ADN ou d'ARN obtenus par synthèse chimique, puis traiter ces segments d'ADN ou d'ARN par des procédés connus en soi de préparation de copies d'ADN (c ADN) bicaténaire en vue du clonage des gènes.

### Criblage ou sélection des souches de cellules-hôtes modifiées

L'étape ultérieure du procédé selon l'invention consiste à examiner les cellules transformées ou transfectées, par sélection ou criblage, en vue d'isoler une ou plusieurs cellules dont l'ADN transformant ou transfectant conduise la synthèse d'un produit de transcription (ARN) ou de traduction (protéine) possédant une propriété souhaitée. Ces propriétés peuvent être, par exemple, enzymatiques, fonctionnelles ou structurelles.

Une des particularités les plus remarquables du procédé selon l'invention est de permettre le criblage ou la sélection simultanée d'un produit exploitable (ARN ou protéine) et de son gène producteur. De surcroît, l'ADN synthétisé et cloné comme décrit peut être sélectionné ou criblé en vue d'isoler des séquences d'ADN constituant un produit en soi, doté de propriétés biochimiques exploitables.

On va maintenant décrire, à titre d'exemples non limitatifs, des modes opératoires préférentiels pour le criblage ou la sélection de souches de cellules transformées ainsi que des protéines nouvelles présentant un intérêt en vue d'applications industrielles ou médicales.

Il est à remarquer que, lors de la description des modes opératoires qui précèdent, un certain nombres de manières de procéder à la sélection et au criblage ont été décrits. Tous ces modes opératoires impliquent la purification d'une protéine particulière à partir d'une souche transformée. Ces purifications de protéines peuvent être effectuées conformément aux méthodes usuelles et font appel, en particulier, aux techniques de chromatographie sur gel, sur échangeur d'ion, et à la chromatographie d'affinité. Par ailleurs, les protéines issues de gènes stochastiques peuvent avoir été clonées sous forme de protéines hybrides, comportant, par exemple, une séquence de l'enzyme β-galactosidase permettant la chromatographie d'affinités sur anticorps anti-β-galactosidase et permettant le clivage subséquent de la partie hybride (c'est-à-dire permettant de séparer la partie nouvelle de la partie bactérienne).On va maintenant décrire le principe et la mise en oeuvre de la sélection des peptides ou polypeptides et des gènes correspondants, exprimant ces peptides ou polypeptides, conformément à une deuxième méthode de criblage ou sélection fondée sur la détection de l'aptitude de ces peptides ou polypeptides à catalyser une réaction spécifique.

A titre d'exemple concret non limitatif, on va décrire la mise en oeuvre du criblage ou de la sélection dans le cas particulier de protéines capables de catalyser le clivage du lactose, ce qui est normalement une fonction remplie par l'enzyme β-galactosidase (β-gal).

Comme décrit ci-dessus, la première étape du procédé consiste à engendrer un très grand ensemble de vecteurs d'expression exprimant chacun une protéine nouvelle distincte. De manière concrète, on peut, par exemple, choisir le vecteur d'expression pUC8 avec clonage de séquences stochastiques d'ADN au site de restriction Pst I.
Les plasmides ainsi obtenus sont ensuite introduits dans une souche de E. coli dans le génome de laquelle on a éliminé par les méthodes génétiques conventionnelles le gène naturel pour la β-galactosidase, Z, et un deuxième gène EBG, sans rapport avec le le premier, mais capable de muter vers la fonction β-gal. De telles cellules-hôtes (Z⁻, EBG⁻) ne sont pas capables par elles-mêmes de catalyser l'hydrolyse du lactose et, par conséquent, d'utiliser le lactose comme source de carbone pour la croissance. Ceci permet d'utiliser cette souche hôte pour le criblage ou la sélection de la fonction β-gal.

Une méthode d'essai biologique qui convient pour l'étude des souches transformées de E. coli présentant des gènes nouveaux exprimant la fonction β-gal consiste dans la culture des bactéries ainsi transformées dans des boîtes de Pétri renfermant le milieu X-gal. Dans ce cas, toute colonie bactérienne exprimant une fonction β-gal est visualisée sous forme d'une colonie bleue. En utilisant un tel essai biologique, on peut détecter même une activité catalytique faible. L'activité spécifique des enzymes les plus caractéristiques s'établit entre 10 et 10 000 molécules de produit par seconde.

En supposant qu'une protéine synthétisée par un gène stochastique présente une activité spécifique faible, de l'ordre de une molécule par 100 secondes, il serait toujours possible de détecter une telle activité catalytique. Dans une boîte de Pétri renfermant le milieu X-gal, en présence de l'inducteur non métabolisable IPTG (isopropyl-D-thiogalactoside) la visualisation d'une région bleue requiert le clivage d'environ 10¹⁰ à 10¹¹ molécules de X-gal par millimètre carré. Une colonie bactérienne exprimant un enzyme faible et occupant une superficie de 1mm² contient environ 10⁷ à 10⁸ cellules. Si chaque cellule présente une seule copie de l'enzyme faible, chaque cellule doit catalyser entre 10 000 et 100 clivages de X-gal afin de pouvoir être détectée, ce qui prend environ 2,7 à 270 heures. Etant donné que, dans des conditions sélectives, on peut s'attendre à une amplification du nombre des copies de plasmides par cellules, par exemple de 5 à 20 copies par cellule et même de 100 à 1000, et compte tenu du fait que jusqu'à 10% des protéines de la cellule peuvent être spécifiées par le nouveau gène, la durée nécessaire à la détection d'une colonie bleue dans le cas de 100 molécules d'enzymes de faible activité spécifique par cellule serait de l'ordre de 0,27 heure à 2,7 heures.

Par conséquent, le criblage d'un grand nombre de colonies bactériennes indépendantes, exprimant chacun un gène nouveau différent, en prenant comme critère de sélection la faculté d'exprimer la fonction β-gal est parfaitement réalisable. On peut ainsi réaliser le criblage d'environ 2000 colonies dans une boite de Pétri ordinaire ayant 10 cm de diamètre. Par conséquent, on peut cribler environ 20 millions de colonies sur une feuille d'agar X-gal de 1 m².

Il est à remarquer que les colonies bactériennes apparaissant en bleu sur les boites de Pétri X-gal pourraient être faussement positives en raison d'une mutation dans le gènome bactérien lui conférant la capacité de métaboliser le lactose, ou pour d'autre raisons que celles qui résultent de l'activité catalytique de la protéine nouvelle exprimée par les cellules de la colonie. De tels faux positifs peuvent être directement éliminés en purifiant l'ADN des vecteurs d'expression provenant des colonies positives et en retransformant les cellules-hôtes E. Coli Z⁻,EBG⁻. Si l'activité β-gal résulte de la nouvelle protéine codée par le nouveau gêne dans le vecteur d'expression, toutes les cellules transformées par ce vecteur devraient présenter la fonction β-gal. Au contraire, si la colonie bleue initiale résulte d'une mutation dans le génome de la cellule hôte, il s'agit d'un événement exceptionnel indépendant de la transformation et le nombre des cellules de la nouvelle souche transformée E. Coli servant d'hôte susceptible d'exprimer la fonction β-gal devrait être petit ou nul.

On insistera particulièrement sur la puissance de la purification en masse simultanée des vecteurs d'expression pour tous les clones positifs (bleus) suivie par la retransformation des bactéries naïves. Supposons que l'on désire effectuer un criblage en vue de sélectionner des protéines ayant une fonction catalytique et que la probabilité qu'un nouveau peptide ou polypeptide remplisse cette fonction, au moins faiblement, soit de 10⁻⁶, alors que la probabilité que la souche microbienne hôte E. Coli est sujette à une mutation ayant pour effet qu'elle devienne capable de remplir cette fonction, soit de 10⁻⁵, on peut calculer que sur 20 millions de bactéries transformées soumises au criblage, 20 clones positifs seront, en moyenne, attribuables aux gènes nouveaux sur les vecteurs d'expression que chacune comporte, alors que 200 clones positifs résulteront de mutations d'arrière-plan. La purification en masse des vecteurs d'expression de l'intégralité des 220 clones bactériens positifs et la retransformation des bactéries naïves avec les vecteurs d'expression mis en commun produira un grand nombre de clones positifs constitués de toutes les bactéries transformées avec les 20 vecteurs d'expression qui codent les nouvelles protéines ayant la fonction désirée, et un très petit nombre de clones bactériens résultant de mutations d'arrière-plan contenant les 200 vecteurs d'expression restant sans intérêt. Un petit nombre de cycles de purification de vecteurs d'expression dans les colonies bactériennes positives, ainsi que de retransformation, permet la détection des très rares vecteurs d'expression véritablement positifs par rapport à une activité catalytique désirée, malgré un bruit de fond très élevé de mutations des cellules-hôtes pour cette fonction.

A la suite d'opérations de criblage de ce genre, on peut purifier la nouvelle protéine grâce à la mise en oeuvre de techniques usuelles. La production de cette protéine en grande quantité est rendue possible grâce au fait que l'identification de la protéine utile s'accompagne de l'identification simultanée du gêne codant cette protéine. Par conséquent, on peut utiliser le vecteur d'expression lui-même ou bien on peut transplanter le gène nouveau dans un vecteur d'expression plus approprié à la synthèse et à l'isolation en grande quantité.
On peut appliquer des modes de criblage de ce genre à toute fonction enzymatique pour laquelle il existe un essai biologique approprié. De tels criblages ne nécessitent pas que la fonction enzymatique que l'on recherche soit profitable à la cellule hôte. On peut effectuer un criblage non seulement par rapport à une fonction enzymatique mais également pour toute autre propriété désirée pour laquelle on peut établir un essai biologique approprié. On peut ainsi effectuer même dans le cas simple de la fonction β-gal visualisée sur boîte de Pétri à milieu X-gal, le criblage d'un nombre de gènes nouveaux de l'ordre de 100 millions ou même d'un milliard pour une activité catalytique ou une autre propriété désirée.

### Sélection des cellules-hôtes modifiées

D'autre part, on peut utiliser des techniques de sélection pour toute propriété, catalytique ou autre, dont la présence ou l'absence peut être rendue essentielle à la survie des cellules-hôtes contenant les vecteurs d'expression codant les gènes nouveaux ou pouvant être utiles pour la sélection de virus codant et exprimant le gène nouveau. A titre d'exemple concret, non limitatif, on va maintenant revenir sur la description du mode de sélection en relation avec la fonction β-galactosidase. Une souche appropriée Z⁻EBG⁻ d'E. Coli ne peut pas croître en utilisant le lactose comme seule source de carbone. Ainsi, après la mise en oeuvre de la première étape décrite ci-dessus, on peut cultiver un très grand nombre d'hôtes transformés par des vecteurs d'expression codant les gènes nouveaux, la culture étant effectuée dans des conditions sélectives, soit en diminuant progressivement la concentration des autres sources de carbone, soit en utilisant seulement le lactose dès le départ. Au cours d'une telle sélection. la mutagénèse in vivo par voie de recombinaison ou par récupération explicite de vecteurs d'expression et mutagénèse in vitro de leurs gènes nouveaux par des mutagènes variés, ou par tout autre technique usuelle, permet des améliorations adaptatives de l'aptitude à remplir la fonction catalytique désirée. Lorsqu'il existe à la foi des techniques de sélection et des techniques commodes d'essai biologique, comme dans le cas du présent exemple, on peut utiliser initialement des techniques de sélection pour enrichir la représentation en bactéries hôte exprimant la fonction β-gal, puis effectuer un criblage sur boîte de Pétri à milieu X-gal afin d'identifier efficacement des cellules positives. En l'absence de technique d'essai biologique convenable, l'application de conditions de sélection de plus en plus rigoureuses constitue la voie la plus facile pour purifier un type ou un petit nombre de types de cellules-hôtes distinctes dont les vecteurs d'expression codent les protéines catalysant la réaction choisie.

On peut utiliser ces techniques pour trouver de nouvelles protéines ayant une grande variété de caractéristiques structurelles ou fonctionnelles en plus de l'aptitude à catalyser des réactions spécifiques.

Par exemple, dans le cas de E. Coli une souche mutante du répresseur de l'opéron lactose (i-) exprime de manière constitutive la fonction β-gal en raison du fait que l'opérateur lactose n'est pas réprimé. Toutes les cellules de ce genre produisent des clones bleus sur les boîtes de Pétri contenant le milieu X-gal. Il est possible de transformer de telles souches hôtes avec des vecteurs d'expression synthétisant des protéines nouvelles et effectuer un criblage sur boîte de Pétri à milieu X-gal en vue de détecter les clones qui ne sont pas bleus. Parmi ceux-ci, certains représentent des cas où la nouvelle protéine se fixe sur l'opérateur lactose et réprime la synthèse de β-gal. On peut purifier en masse de tels plasmides, les retransformer , isoler les clones qui ne produisent pas la β-gal et effectuer ensuite une vérification détaillée.

Comme il est mentionné plus haut, le procédé peut être utilisé aux fins de créer puis d'isoler non seulement des protéines exploitables mais aussi des ARN et des ADN constituant des produits en soi, dotés de propriétés exploitables. Ceci résulte évidemment du fait que d'une part, le procédé consiste à créer des séquences stochastiques d'ADN, susceptibles d'interagir directement avec d'autres constituants cellulaires ou biochimiques, et que d'autre part ces séquences clonées dans un vecteur d'expression sont transcrites en ARN eux aussi capables de multiples interactions biochimiques.

### Création et sélection d'un ARN exploitable en soi

Soit un grand nombre de séquences d'ADN stochastique produites comme il a été décrit et clonées dans un vecteur d'expression. Il va de soi que l'ARN transcrit à partir de ces séquences dans des cellules-hôtes transformées peut être un produit exploitable en soi.
A titre d'exemple non limitatif, on peut sélectionner un gène stochastique codant pour un ARN de transfert (t-ARN) suppresseur par la procédure suivante :
On transforme par un grand nombre ( ≧ 10⁸) de séquences stochastiques, une souche bactérienne compétente comportant une mutation "nonsense" dans le gène arg E. On étale les bactéries transformées sur milieu minimal sans arginine et contenant l'antibiotique de sélection du plasmide (ampicilline s'il s'agit du vecteur pUC8).Seules les bactéries transformées qui sont devenues capables de synthétiser l'arginine pourront croître. Ce phénotype peut résulter soit d'une mutation en retour soit de l'introduction dans la cellule d'un suppresseur. Il est facile de tester chaque colonie transformée pour déterminer si le phénotype arg⁺ résulte ou non de la présence du gène stochastique dans son vecteur : on se contente de préparer le plasmide de cette colonie et de vérifier qu'il confère le phénotype Arg⁺ à toute cellule arg E qu'il transforme.

## Revendications

1. Procédé de production de peptides, polypeptides ou protéines ayant au moins une propriété donnée, selon lequel on fournit simultanément une pluralité de gènes, au sein d'un même milieu, de façon à produire une bibliothèque de gènes, on amplifie les gènes, on effectue un criblage et/ou une sélection, de façon à identifier au moins un gène capable d'être exprimé sous forme de peptide, polypeptide ou protéine ayant ladite propriété, et l'on produit au moins un peptide, polypeptide ou protéine en utilisant l'information génétique ainsi obtenue, caractérisé en ce que les gènes sont, au moins partiellement, composés de polynucléotides synthétiques stochastiques.

2. Procédé selon la revendication 1, caractérisé en ce que l'on effectue le criblage et/ou la sélection sur ladite bibliothèque de gènes.

3. Procédé selon la revendication 1, caractérisé en ce qu'il comprend, en outre, l'introduction desdits gènes dans des cellules-hôtes, de façon à produire une bibliothèque de cellules-hôtes modifiées renfermant ces gènes, et en ce que l'on effectue le criblage et/ou la sélection sur la bibliothèque de cellules-hôtes modifiées ainsi obtenue.

4. Procédé selon la revendication 3, selon lequel on cultive simultanément les souches indépendantes de cellules-hôtes modifiées, de manière à cloner les gènes stochastiques et à provoquer la production des protéines exprimées par chacun de ces gènes stochastiques, on effectue le criblage et/ou la sélection des souches de cellules-hôtes modifiées, de façon à identifier les souches produisant des peptides, polypeptides ou protéines ayant ladite propriété donnée, on isole les souches ainsi identifiées et on les cultive de façon à produire au moins un peptide ou polypeptide, ou au moins une protéine ayant cette propriété.

5. Procédé selon l'une des revendications 1 à 4, caractérisé par le fait qu'on produit les gènes par copolymérisation stochastique des quatre sortes de deoxyphosphonucléotides A, C, G et T à partir des deux extrémités d'un vecteur d'expression préalablement linéarisé, puis formation d'extrémités cohésives de façon à former un premier brin d'ADN stochastique, constitué d'une molécule du vecteur d'expression possédant deux séquences stochastiques dont les extrémités 3' sont complémentaires, suivie de la synthèse du second brin de cet ADN stochastique.

6. Procédé selon l'une des revendications 1 à 4, caractérisé par le fait qu'on produit les gènes par copolymérisation stochastique d'oligonucléotides bicaténaire sans extrémités cohésives, de façon à former des fragments d'ADN stochastiques, suivie de la ligation de ces fragments à un vecteur d'expression préalablement linéarisé.

7. Procédé selon la revendication 5 ou la revendication 6, caractérisé par le fait que le vecteur d'expression est un plasmide.

8. Procédé selon la revendication 7, caractérisé par le fait que le vecteur d'expression est le plasmide pUC8.

9. Procédé selon la revendication 5 ou la revendication 6, caractérisé par le fait que le vecteur d'expression est un fragment d'ADN viral.

10. Procédé selon la revendication 5 ou la revendication 6, caractérisé par le fait que le vecteur d'expression est un hybride de plasmide et d'ADN viral.

11. Procédé selon la revendication 5 ou la revendication 6, caractérisé par le fait que le vecteur d'expression est un phage.

12. Procédé selon l'une des revendications 3 à 11, caractérisé par le fait que les cellules-hôtes sont des cellules procaryotes.

13. Procédé selon l'une des revendications 3 à 11, caractérisé par le fait que les cellules-hôtes sont des cellules eucaryotes.

14. Procédé selon la revendication 12, caractérisé par le fait que les cellules-hôtes sont choisies parmi HB 101 et C 600.

15. Procédé selon la revendication 6, caractérisé par le fait que les oligonucléotides forment un groupe d'octamères palindromiques.

16. Procédé selon la revendication 15, caractérisé par le fait que le groupe d'octamères palindromiques est le groupe suivant:
5' GGAATTCC 3'
5' GGTCGACC 3'
5' CAAGCTTG 3'
5' CCATATGG 3'
5' CATCGATG 3'

17. Procédé selon la revendication 6, caractérisé par le fait que les oligonucléotides forment un groupe d'heptamères palindromiques.

18. Procédé selon la revendication 17, caractérisé par le fait que le groupe d'heptamères palindromiques est le groupe suivant :
5' XTCGCGA 3'
5' XCTGCAG 3'
5' RGGTACC 3'
où X = A, G, C ou T et R = A ou T.

19. Procédé selon la revendication 7 et l'une des revendications 15 ou 17, caractérisé par le fait que l'on isole et purifie l'ADN transformant des plasmides provenant d'une culture de souches indépendantes de cellules-hôtes modifiées obtenues en procédant de la manière spécifiée dans la revendication 15 ou la revendication 17, puis que l'on provoque la coupure de l'ADN purifié au moyen d'au moins un enzyme de restriction correspondant à un site spécifique de coupure enzymatique présent dans ces octamères ou heptamères palindromiques mais absent du vecteur d'expression utilisé, cette coupure étant suivie de l'inactivation de l'enzyme de restriction, et que l'on traite ensuite simultanément l'ensemble des fragments d'ADN stochastiques linéarisés ainsi obtenus par la T4 DNA ligase, de façon à créer un nouvel ensemble d'ADN contenant des séquences stochastiques nouvelles, et que l'on utilise ce nouvel ensemble d'ADN transformant pour modifier des cellules-hôtes et cloner les gènes et, finalement, que l'on crible et/ou sélectionne et isole les nouvelles souches de cellules-hôtes transformées et qu'on les cultive de façon à produire au moins un peptide ou polypeptide.

20. Procédé selon l'une des revendications 1 à 19, caractérisé par le fait que ladite propriété est l'aptitude à catalyser une réaction chimique donnée.

21. Procédé selon l'une des revendications 1 à 19, caractérisé par le fait que ladite propriété est l'aptitude à modifier sélectivement l'activité catalytique d'un polypeptide.

22. Procédé selon l'une des revendications 1 à 19, caractérisé par le fait que ladite propriété est l'aptitude à se lier à un ligand donné.

23. Procédé selon les revendications 3 et 10, caractérisé par le fait que l'on identifie et isole les souches de cellules-hôtes modifiées produisant les peptides ou polypeptides ayant la propriété désirée par chromatographie d'affinité sur anticorps correspondant à une protéine exprimée par la partie naturelle de l'ADN hybride.

24. Procédé selon la revendication 23, caractérisé par le fait que la partie naturelle de l'ADN hybride contient un gène exprimant la β-galactosidase, et que l'on identifie et isole lesdites souches de cellules-hôtes modifiées par chromatographie d'affinité sur anticorps anti-β-galactosidase.

25. Procédé selon la revendication 23 ou la revendication 24, caractérisé par le fait qu'après expression et purification des peptides ou polypeptides hybrides, on sépare et isole leurs parties nouvelles.

26. Procédé selon l'une des revendications 3 à 11, 13 et 15 à 19, caractérisé par le fait que les cellules-hôtes consistent en bactéries du genre Escherichia coli dont le génome ne contient ni le gène naturel exprimant la β-galactosidase, ni le gène EBG, c'est-à-dire des bactéries E. coli (Z⁻, EBG⁻), que l'on cultive les cellules-hôtes modifiées en présence du milieu X-gal et de l'inducteur IPTG, que l'on détecte, dans le milieu de culture, les clones positifs pour la fonction β-galactosidase et qu'on transplante ensuite cet ADN dans une souche de cellules-hôtes appropriée à la culture en grande quantité en vue de la production industrielle d'au moins un peptide, polypeptide ou protéine.

27. Procédé selon l'une des revendications 1 à 19, caractérisé par le fait que ladite propriété est l'aptitude à se lier à un composé donné.

28. Procédé selon la revendication 27, caractérisé par le fait que ledit composé est choisi parmi les peptides, les polypeptides et les protéines.

29. Procédé selon la revendication 27, caractérisé par le fait que ledit composé est choisi parmi les séquences d'ADN et d'ARN.

30. Procédé de production d'ADN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on cultive simultanément les souches indépendantes de cellules-hôtes modifiées ainsi produites, que l'on effectue un criblage et/ou une sélection de cet ensemble, de façon à identifier les cellules-hôtes qui renferment dans leur génomes des séquences stochastiques d'ADN présentant au moins une propriété désirée et que l'on isole l'ADN à partir de cultures de cellules-hôtes ainsi identifiées.

31. Procédé selon la revendication 30, caractérisé par le fait que ladite propriété est l'aptitude à se lier à un composé donné.

32. Procédé selon la revendication 31, caractérisé par le fait que ledit composé est choisi parmi les peptides, les polypeptides et les protéines.

33. Procédé de production d'ARN, caractérisé par le fait que l'on produit simultanément, au sein d'un même milieu, des gènes au moins partiellement composés de polynucléotides synthétiques stochastiques, que l'on introduit les gènes ainsi obtenus dans des cellules-hôtes, de façon à produire un ensemble de cellules-hôtes modifiées, que l'on cultive simultanément les souches indépendantes de cellules-hôtes modifiées ainsi produites, que l'on effectue un criblage et/ou une sélection de cet ensemble, de façon à identifier les cellules-hôtes qui renferment des séquences stochastiques d'ARN présentant au moins une propriété désirée et que l'on isole l'ARN à partir de cultures de cellules-hôtes ainsi identifiées.

34. Procédé selon la revendication 33, caractérisé par le fait que ladite propriété est l'aptitude à se lier à un composé donné.

35. Procédé selon la revendication 33, caractérisé par le fait que ladite propriété est l'aptitude à catalyser une réaction chimique donnée.

36. Procédé selon la revendication 33, caractérisé par le fait que ladite propriété est d'avoir la fonction d'un ARN de transfert.

37. Procédé de production de peptides, polypeptides ou protéines ayant au moins une propriété donnée, selon lequel on produit une bibliothèque de gènes, on amplifie et on traduit ces gènes de façon à produire des peptides ou polypeptides ou des protéines exprimés par au moins l'un des gènes de cette bibliothèque, et l'on effectue un criblage et/ou une sélection de façon à identifier au moins un peptide ou polypeptide ou au moins une protéine ayant ladite propriété, caractérisé en ce que les gènes sont, au moins partiellement, composés de polynucléotides synthétiques stochastiques.

## Claims

1. Process for the production of peptides, polypeptides or proteins having at least one predetermined property, comprising the steps of simultaneously providing genes in a common medium, so as to produce a gene library, amplifying the genes, carrying out screening and/or selection, so as to identify at least one gene capable of being expressed as a peptide, polypeptide or protein having said property; and producing at least one peptide, polypeptide or protein by a method using the thus obtained genetic information, characterized in that the genes consist, at least partially, of stochastic synthetic polynucleotides.

2. Process according to claim 1, characterized in that the step of carrying out screening and/or selection is effected on the said gene library.

3. Process according to claim 1, characterized in that it further comprises the step of introducing said genes into host cells, so as to form a library of transformed host cells corresponding to said gene library, and in that the step of carrying out screening and/or selection is effected on the thus obtained transformed host cells library.

4. Process according to claim 3, according to which the independent strains of transformed host cells are simultaneously cultivated, so as to clone the stochastic genes and lead to the production of proteins expressed by each of these stochastic genes, screening and/or selection of the strains of transformed host cells is carried out so as to identify the strains producing peptides, polypeptides or proteins having said predetermined property, the thus identified strains are isolated and grown in a manner so as to produce at least a peptide or polypeptide, or at least a protein having said property.

5. Process according to one of claims 1 to 4, characterized by the fact that the genes are produced by stochastic copolymerization of the four types of deoxyphosphonucleotides A, C, G and T, starting from the two extremities of an expression vector which was previously linearized, then by formation of cohesive extremities to create a first strand of stochastic DNA constituted of a molecule of expression vector possessing two stochastic sequences whose 3' extremities are complementary, followed by synthesis of the second strand of the stochastic DNA.

6. Process according to one of claims 1 to 4, characterized in the fact that the genes are produced by stochastic copolymerization of double stranded oligonucleotides which do not have cohesive ends, in a manner so as to form fragments of stochastic DNA, followed by ligation of these fragments in an expression vector which was previously linearized.

7. Process according to claim 5 or 6, characterized by the fact that the expression vector is a plasmid.

8. Process according to claim 7, characterized by the fact that the plasmid is pUC8.

9. Process according to claim 5 or claim 6, characterized by the fact that the expression vector is a fragment of viral DNA.

10. Process according to claim 5 or claim 6, characterized by the fact that the expression vector is a hybrid of plasmid and viral DNA.

11. Process according to claim 5 or claim 6, characterized by the fact that the expression vector is a phage.

12. Process according to claims 3 to 11, characterized by the fact that the host cells are prokaryotic cells.

13. Process according to claims 3 to 11, characterized by the fact that the host cells are eukaryotic cells.

14. Process according to claim 12 characterized by the fact that the host cells are chosen among HB101 and C600.

15. Process according to claim 6 characterized by the fact that the oligonucleotides form a group of palindromic octamers.

16. Process according to claim 15, characterized by the fact that the group of palindromic octamers is the following group:
5' GGAATTCC 3'
5' GGTCGACC 3'
5' CAAGCTTG 3'
5' CCATATGG 3'
5' CATCGATG 3'

17. Process according to claim 6, characterized by the fact that the oligonucleotides form a group of palindromic heptamers.

18. Process according to claim 17, characterized by the fact that the group of palindromic heptamers is the following group:
5' XTCGCGA 3'
5' XCTGCAG 3'
5' RGGTACC 3'
where X = A, G, C or T, and R = A or T.

19. Process according to claim 7 and one of the claims 15 or 17, characterized by the fact that one first isolates and purifies the transforming DNA derived from a culture of independent clones of transformed host cells obtained by proceeding in the manner specified in claim 15 or in claim 17, then that one cuts the purified DNA by means of at least one restriction enzyme which corresponds to a specific restriction site present in the palindromic octamers or heptamers, but absent from the expression vector being utilized, that one thereafter simultaneously treats the ensemble of linearized stochastic DNA fragments so obtained by T4 DNA ligase in such a manner as to create a new ensemble of DNA containing new stochastic sequences, and that one uses this new ensemble of transforming DNA to transform host cells and clone such genes, and finally that one carries out screening and/or selection and isolates the new clones of transformed cells and that one grows these so as to produce at least one peptide, polypeptide or protein.

20. Process according to one of claims 1 to 19 characterized by the fact that the said property is the capacity to catalyse a given chemical reaction.

21. Process according to one of claims 1 to 19, characterized by the fact that the said property is the capacity to modify selectively the catalytic activity of a polypeptide.

22. Process according to one of claims 1 to 19, characterized by the fact that the said property is the capacity to bind to a given ligand.

23. Process according to claims 3 and 10, characterized by the fact that strains of transformed host cells producing peptides or polypeptides having the desired property are identified and isolated by affinity chromatography on antibodies corresponding to a protein expressed by the natural fragment of the DNA hybrid.

24. Process according to claim 23, characterized by the fact that the natural fragment of the DNA hybrid contains a gene expressing β-galactosidase, and that one identifies and isolates the said strains of transformed cell hosts by affinity chromatography with anti-β-galactosidase antibodies.

25. Process according to claim 23 or claim 24, characterized by the fact that after expression and purification of the hybrid peptides or polypeptides, the novel fragments are separated and isolated.

26. Process according to one of claims 3 to 11, 13 and 15 to 19, characterized by the fact that the host cells consist in bacteria of Escherichia coli type, whose genome contains neither the natural β-galactosidase gene, nor the EBG gene, that is Z⁻, EGB⁻ E coli, and that these transformed host cells are cultured in an X-gal medium also containing the inducer IPTG, that clones which are positive for β-galactosidase function are detected in the culture milieu, that thereafter this DNA is transplanted to a clone of host cells appropriate for industrial production of at least one peptide, polypeptide or protein.

27. Process according to one of claims 1 to 19, characterized by the fact that the said property is the capacity to bind to a given compound.

28. Process according to claim 27, characterized by the fact that the said compound is chosen among the peptides, polypeptides and proteins.

29. Process according to claim 27, characterized by the fact that the said compound is chosen among the sequences of DNA and RNA.

30. Process for the production of DNA, characterized by the fact that, in the same milieu, genes which are at least partially composed of stochastic synthetic polynucleotides are produced, that the genes so produced are introduced into host cells in a manner to produce an ensemble of transformed host cells, that these are grown so as to produce independent clones of the host cells so produced, that screening and/or selection is carried out on this ensemble to identify those host cells which contain in their genome those stochastic sequences of DNA having at least one desired property, and that such DNA is isolated from the identified cultures of the host cells.

31. Process according to claim 30, characterized by the fact that the said property is the capacity to bind to a given compound.

32. Process according to claim 31, characterized by the fact that the said compound is chosen among the peptides, polypeptides and proteins.

33. Process for the production of RNA, characterized by the fact that, in the same milieu, genes which are at least partially composed of synthetic stochastic polynucleotides are produced simultaneously, that the genes thus obtained are introduced in host cells in a manner to produce an ensemble of transformed host cells, that the independent clones of transformed host cells so produced are grown simultaneously, that a screening and/or selection is carried out on this ensemble in a manner to identify those host cells containing stochastic sequences of RNA having at least one desired property, and that the RNA is isolated from the cultures of host cells so identified.

34. Process according to claim 33, characterized by the fact that the said property is the capacity to bind to a given compound.

35. Process according to claim 33, characterized by the fact that the said property is the capacity to catalyse a given chemical reaction.

36. Process according to claim 33, characterized by the fact that the said property is to have the function of a transfer RNA.

37. Process for the production of a peptide, polypeptide or protein having at least one predetermined property, comprising the steps of: producing a library of genes, amplifying and translating these genes to produce peptides, polypeptides or proteins expressed by at least one of the genes of said library and carrying out screening and/or selection methods to identify at least one peptide, polypeptide or protein having said property, characterized in that the genes are, at least partially, made of stochastic synthetic polynucleotide sequences.

## Patentansprüche

1. Verfahren zur Herstellung von Peptiden, Polypeptiden oder Proteinen mit mindestens einer bestimmten Eigenschaft, wobei man gleichzeitig eine Vielzahl von Genen in einem gemeinsamen Milieu hinzufügt, um eine Genenbibliothek zu produzieren, man die Gene multipliziert, man ein Screening und/oder eine Selektion durchführt, um mindestens ein Gen zu identifizieren, das fähig ist, als Peptid, Polypeptid oder Protein mit der erwähnten Eigenschaft exprimiert zu werden, und man mindestens ein Peptid, Polypeptid oder Protein herstellt, indem man die so erhaltene genetische Information benutzt, dadurch gekennzeichnet, dass die Gene zumindest teilweise aus stochastischen, synthetischen Polynukleotiden zusammengesetzt sind.

2. Verfahren nach Anspruch 1, dadurch gekennzeichnet, dass man das Screening und/oder die Selektion auf der erwähnten Genenbibliothek durchführt.

3. Verfahren nach Anspruch 1, dadurch gekennzeichnet, dass man ferner die erwähnten Gene in Wirtszellen einbringt, um eine Bibliothek von transformierten Wirtszellen zu produzieren, welche diese Gene enthalten, und dass man das Screening und/oder die Selektion auf der so erhaltenen Bibliothek von transformierten Wirtszellen durchführt.

4. Verfahren nach Anspruch 3, wobei man die unabhängigen Klone der transformierten Wirtszellen gleichzeitig kultiviert, um die stochastischen Gene zu klonieren und die Bildung von Proteinen, die durch jedes dieser stochastischen Gene exprimiert werden, zu bewirken, man das Screening und/oder die Selektion an solchen Klonen transformierter Wirtszellen durchführt, um die Klone zu identifizieren, die Peptide, Polypeptide oder Proteine mit der erwähnten bestimmten Eigenschaft bilden, und man die so identifizierten Klone isoliert, um mindestens ein Peptid oder Polypeptid oder mindestens ein Protein mit dieser Eigenschaft zu produzieren.

5. Verfahren nach einem der Ansprüche 1 bis 4, dadurch gekennzeichnet, dass die Gene durch stochastische Copolymerisation der vier Arten von Deoxyphosphonukleotiden A, C, G und T gebildet werden, ausgehend von den zwei Enden eines vorher linearisierten Expressionsvektors, dann durch Bildung kohäsiver Enden, um einen ersten Strang von stochastischer DNS zu bilden, der aus einem Molekül des Expressionsvektors aufgebaut ist, der zwei stochastische Sequenzen besitzt, dessen 3'-Enden komplementär sind, gefolgt von der Synthese des zweiten Stranges der stochastischen DNS.

6. Verfahren nach einem der Ansprüche 1 bis 4, dadurch gekennzeichnet, dass die Gene durch stochastische Copolymerisation von doppelsträngigen Oligonukleotiden, die keine kohäsiven Enden besitzen, in einer solchen Weise hergestellt werden, um Fragmente von stochastischer DNS zu bilden, gefolgt von der Ligierung dieser Fragmente in einem vorher linearisierten Expressionsvektor.

7. Verfahren nach Anspruch 5 oder Anspruch 6, dadurch gekennzeichnet, dass der Expressionsvektor ein Plasmid ist.

8. Verfahren nach Anspruch 7, dadurch gekennzeichnet, dass der Expressionsvektor das Plasmid pUC8 ist.

9. Verfahren nach Anspruch 5 oder Anspruch 6, dadurch gekennzeichnet, dass der Expressionsvektor ein Fragment einer viralen DNS ist.

10. Verfahren nach Anspruch 5 oder Anspruch 6, dadurch gekennzeichnet, dass der Expressionsvektor ein Hybrid eines Plasmids und einer viralen DNS ist.

11. Verfahren nach Anspruch 5 oder Anspruch 6, dadurch gekennzeichnet, dass der Expressionsvektor ein Phage ist.

12. Verfahren nach einem der Ansprüche 3 bis 11, dadurch gekennzeichnet, dass die Wirtszellen prokaryontische Zellen sind.

13. Verfahren nach einem der Ansprüche 1 bis 7, dadurch gekennzeichnet, dass die Wirtszellen eukaryontische Zellen sind.

14. Verfahren nach Anspruch 12, dadurch gekennzeichnet, dass die Zellen ausgewählt sind aus HB101 und C600.

15. Verfahren nach Anspruch 6, dadurch gekennzeichnet, dass die Oligonukleotide eine Gruppe palindromischer Octamerer bilden.

16. Verfahren nach Anspruch 15, dadurch gekennzeichnet, dass die Gruppe palindromischer Octamerer die folgende Gruppe ist:
5' GGAATTCC 3'
5' GGTCGACC 3'
5' CAAGCTTG 3'
5' CCATATGG 3'
5' CATCGATG 3'

17. Verfahren nach Anspruch 3, dadurch gekennzeichnet, dass die Oligonukleotide eine Gruppe palindromischer Heptamerer bilden.

18. Verfahren nach Anspruch 17, dadurch gekennzeichnet, dass die Gruppe palindromischer Heptamere die folgende Gruppe ist:
5' XTCGCGA 3'
5' XCTGCAG 3'
5' RGGTACC 3'
worin X = A, G, C oder T, und R = A oder T.

19. Verfahren nach Anspruch 7 und einem der Ansprüche 15 oder 17, dadurch gekennzeichnet, dass man zuerst die von einer Kultur unabhängiger Klone transformierter Wirtszellen abgeleitete transformierende DNS reinigt, die erhalten wird durch die in Anspruch 15 oder in Anspruch 17 angegebene Verfahrensweise, dann die gereinigte DNS mittels mindestens einem Restriktionsenzym, das einer spezifischen Restriktionsstelle entspricht, welche in diesen palindromischen Octameren oder Heptameren vorhanden ist, aber in dem verwendeten Expressionsvektor nicht vorhanden ist, schneidet und dann das Restriktionsenzym inaktiviert wird, dass man danach das so erhaltene Ensemble linearisierter stochastischer DNS-Fragmente mit T4 DNS-Ligase in einer solchen Weise behandelt, um ein neues Ensemble von DNS, das neue stochastische Sequenzen enthält, zu schaffen, und dass man dieses neue Ensemble transformierender DNS verwendet, um Wirtszellen zu transformieren und solche Gene zu klonen, und dass man schliesslich das Screening und/oder eine Selektion durchführt und die neuen Klone transformierter Zellen isoliert, und dass man diese vermehrt, um mindestens ein Peptid oder Polypeptid herzustellen.

20. Verfahren nach einem der Ansprüche 1 bis 19, dadurch gekennzeichnet, dass die Eigenschaft die Fähigkeit ist, eine bestimmte chemische Reaktion zu katalysieren.

21. Verfahren nach einem der Ansprüche 1 bis 19, dadurch gekennzeichnet, dass die Eigenschaft die Fähigkeit ist, selektiv die katalytische Aktivität eines Polypeptids zu modifizieren.

22. Verfahren nach einem der Ansprüche 1 bis 19, dadurch gekennzeichnet, dass die Eigenschaft die Fähigkeit ist, an einen bestimmten Liganden zu bilden.

23. Verfahren nach Anspruch 3 und 10, dadurch gekennzeichnet, dass Klone transformierter Wirtszellen, die Peptide oder Polypeptide mit der gewünschten Eigenschaft produzieren, durch Affinitätschromatographie an Antikörpern, die einem Protein entsprechen, das durch das natürliche Fragment des DNS-Hybrids exprimiert ist, identifiziert und isoliert werden.

24. Verfahren nach Anspruch 23, dadurch gekennzeichnet, dass das natürliche Fragment des DNS-Hybrids ein Gen enthält, das β-Galactosidase exprimiert, und dass man die Klone der transformierten Wirtszellen durch Affinitätschromatographie mit Anti-β-Galactosidase-Antikörpern identifiziert und isoliert.

25. Verfahren nach Anspruch 23 oder Anspruch 24, dadurch gekennzeichnet, dass nach Expression und Reinigung der Hybridpeptide oder -polypeptide die neuen Fragmente abgetrennt und isoliert werden.

26. Verfahren nach einem der Ansprüche 3 bis 11, 13 und 15 bis 19, dadurch gekennzeichnet, dass die Wirtszellen aus Bakterien des Escherichia coli-Typs bestehen, dessen Genom weder das natürliche β-Galactosidase-Gen, noch das EBG-Gen enthält, d.h. Z⁻, EBG⁻, E. coli, und dass diese transformierten Wirtszellen in einem X-gal-Medium, das auch den Inducer 1PTG enthält, kultiviert werden, dass Klone, die für β-Galactosidase-Funktion positiv sind, in dem Kulturmedium bestimmt werden, dass danach diese DNS auf einen Klon von Wirtszellen transplantiert wird, der geeignet ist zur industriellen Herstellung von mindestens einem Peptid, Polypeptid oder Protein.

27. Verfahren nach einem der Ansprüche 1 bis 19, dadurch gekennzeichnet, dass die Eigenschaft die Fähigkeit ist, an eine bestimmte Verbindung zu binden.

28. Verfahren nach Anspruch 27, dadurch gekennzeichnet, dass die Verbindung ausgewählt ist aus Peptiden, Polypeptiden und Proteinen.

29. Verfahren nach Anspruch 27, dadurch gekennzeichnet, dass die Verbindung ausgewählt ist unter den Sequenzen von DNS und RNS.

30. Verfahren zur Herstellung von DNS, dadurch gekennzeichnet, dass man einem gleichen Milieu gleichzeitig Gene produziert, die mindestens teilweise aus stochastischen, synthetischen Polynukleotiden zusammengesetzt sind, dass man die so produzierten Gene in die Wirtszellen in einer Weise einführt, um ein Ensemble von transformierten Wirtszellen zu bilden, dass man gleichzeitig die unabhängigen Klone der so hergestellten transformierten Wirtszellen kultiviert, dass man ein Screening und/oder eine Selektion an diesem Ensemble durchführt, um diese Wirtszellen zu identifizieren, die in ihren Genomen stochastische Sequenzen von DNS enthalten, die mindestens eine gewünschte Eigenschaft besitzen, und dass man die DNS aus Kulturen der so identifizierten Wirtszellen isoliert.

31. Verfahren nach Anspruch 30, dadurch gekennzeichnet, dass die Eigenschaft die Fähigkeit ist, an eine bestimmte Verbindung zu binden.

32. Verfahren nach Anspruch 31, dadurch gekennzeichnet, dass die Verbindung ausgewählt ist unter Peptiden, Polypeptiden und Proteinen.

33. Verfahren zur Herstellung von RNS, dadurch gekennzeichnet, dass man in einem gleichen Milieu gleichzeitig Gene produziert, die mindestens teilweise aus synthetischen, stochastischen Polynukleotiden zusammengesetzt sind, dass man die so erhaltenen Gene in Wirtszellen in einer Weise einbringt, um ein Ensemble transformierter Wirtszellen zu produzieren, dass man gleichzeitig die unabhängigen Klone der so hergestellten transformierten Wirtszellen kultiviert, dass man ein Screening und/oder eine Selektion an diesem Ensemble in einer Weise durchführt, um diese Wirtszellen zu identifizieren, die stochastische RNS-Sequenzen enthalten, die mindestens eine gewünschte Eigenschaft besitzen, und dass man die RNS aus Kulturen der so identifizierten Wirtszellen isoliert.

34. Verfahren nach Anspruch 33, dadurch gekennzeichnet, dass die Eigenschaft die Fähigkeit ist, an eine bestimmte Verbindung zu binden.

35. Verfahren nach Anspruch 33, dadurch gekennzeichnet, dass die Eigenschaft die Fähigkeit ist, eine bestimmte chemische Reaktion zu katalysieren.

36. Verfahren nach Anspruch 33, dadurch gekennzeichnet, dass die Eigenschaft daraus besteht, die Funktion einer Transfer-RNS zu haben.

37. Verfahren zur Herstellung von Peptiden, Polypeptiden oder Proteinen mit mindestens einer bestimmten Eigenschaft, wobei man eine Genenbibliothek produziert, man die Gene so amplifiziert und übersetzt, um Peptide oder Polypeptide oder Proteine herzustellen, welche durch mindestens eines der Genen dieser Bibliothek exprimiert werden, und man ein Screening und/oder eine Selektion durchführt, um mindestens ein Peptid oder Polypeptid oder mindestens ein Protein mit dieser Eigenschaft zu identifizieren, dadurch gekennzeichnet, dass die Gene zumindest teilweise aus stochastischen, synthetischen Polynukleotiden zusammengesetzt sind.
